# EUROPEAN PATENT SPECIFICATION

(11) **EP 0 904 064 B1**
(45) Date of publication and mention of the grant of the patent: **24.10.2001**
(21) Application number: 97920854.3
(22) Date of filing: 28.04.1997
(51) Int. Cl.: A61K 9/48, A61K 9/107, A61K 31/565, A61K 31/57

(54) **ORAL PHARMACEUTICAL COMPOSITIONS CONTAINING SEX HORMONES**
ORALE PHARMAZEUTISCHE ZUSAMMENSETZUNGEN MIT SEXUALHORMONEN
PREPARATION PHARMACEUTIQUE ORALE CONTENANT DES HORMONES SEXUELLES

(30) Priority: 26.04.1996 GB 9608719
(43) Date of publication of application: 31.03.1999
(73) Proprietor: R.P. Scherer Technologies, Inc., Paradise Valley, Nevada 89119 (US)
(72) Inventor: PERRY, Elizabeth, Anne, Swindon, Wiltshire SN6 7BJ (GB); CHANDLER, Susan, Gerrard, Pewsey, Wiltshire SN9 5DH (GB); FERDINANDO, Josephine, Joan, Christine, Chippenham, Wiltshire SN15 3XW (GB)
(74) Representative: Pearce, Anthony Richmond
(86) International application number: GB9701247
(87) International publication number: WO9740823

(56) References cited:
- WO-A-95/24893

## Description

This invention relates to pharmaceutical compositions for oral administration and in particular to pharmaceutical compositions comprising sex hormones, such as progesterone, oestradiol and testosterone.

The demand for hormonal preparations to treat menopausal symptoms has been growing rapidly as evidence has accumulated of the benefits of hormone replacement therapy for both symptomatic relief of menopausal symptoms and the prevention of osteoporosis. It is estimated that in the United Kingdom 25% of women suffer from osteoporosis. A preferred treatment for the symptoms and complications of the menopause is a cyclical treatment regimen of an oestrogen alone or a combination of an oestrogen and a progestogen. Most products available, however, contain oestrogens and progestogens from either non-human animal sources or which are synthetic analogues of human hormones.

Progesterone is a naturally occurring female progestogen. Synthetic progestogens have been used for many years as contraceptives and for preventing endometrial hyperplasia in women receiving oestrogens as hormone replacement therapy. Natural progesterone has not been widely used because of its poor oral bioavailability.

Progesterone has traditionally been administered intramuscularly or by the vaginal or rectal route in order to avoid the high rate of "first pass" hepatic metabolism for the drug. Such methods of administration are not universally popular however, and an effective oral dosage form is required. A suspension of micronised progesterone in oil encapsulated in a softgel has recently been available but, as for solid dosage forms, dissolution is still required in vivo and limits the rate of absorption, particularly as the aqueous solubility of progesterone is very low. Gradual dissolution and absorption of progesterone from a suspension provides a steady flow of the drug to the liver where it is extensively metabolised, thereby limiting the amount of the dose reaching the systemic circulation. Increasing the rate of drug absorption beyond the rate of metabolism in the liver would be expected to result in increased oral bioavailability.

Testosterone, generally in the form of a testosterone ester, has also been administered therapeutically, e.g. in hormone replacement treatments. Testosterone has been applied by intramuscular injection, implants and orally, e.g. in the form of capsules.

PCT/US90/00721 discloses pharmaceutical compositions for oral administration comprising a therapeutically effective amount of a pharmaceutical compound, an organic solvent and an oil. A solution formulation comprising ethanol, palm oil, polyethylene glycol fatty acid ester, progesterone and N-methyl-2-pyrrolidine (organic solvent) is disclosed. The formulation exhibits improved bioavailability compared to a formulation of micronised progesterone in peanut oil. However, the high quantity of organic solvent present in the formulation is undesirable for use in softgel capsules as it is likely to cause stability problems.

US-A-4963540 discloses pharmaceutical compositions which may be filled in capsules, comprising micronised progesterone in an oil vehicle which is high in glycerides of polyunsaturated fatty acids. It is stated that micronised progesterone particles suspended in such a vehicle are more readily absorbed into the bloodstream than other types of oral progesterone formulations.

WO95/24893 discloses a carrier system for a hydrophobic drug which comprises a digestible oil and a pharmaceutically acceptable surfactant for dispersing the oil in vivo upon administration of the carrier system, said surfactant comprising a hydrophilic surfactant component, and being such that it does not substantially inhibit the lipolysis of the digestible oil. The surfactant generally comprises:
(a) a hydrophilic surfactant component which substantially inhibits the in vivo lipolysis of said digestible oil, and
(b) a lipophilic surfactant component capable of at least substantially reducing said inhibitory effect of said hydrophilic surfactant component.

The generally preferred range of digestible oil in the carrier system is 10 to 90% with the more preferred range being 20 to 60%, most preferably 25 to 45%.

Several formulations comprising dissolved progesterone are disclosed in which ethanol is present but the maximum concentration of progesterone achieved was 4% by weight. In order to achieve a dose of 50mg of progesterone completely dissolved in the formulation in a softgel capsule it is necessary to employ a large (20 oblong) capsule size or divide the dose into two smaller capsules. Neither of these options is conducive to patient compliance.

It has now been found that particular combinations of digestible oil and mixtures of lipophilic and hydrophilic surfactants provide a carrier system which is capable of solubilising significant amounts of hydrophobic drugs, such as, progesterone.

According to one aspect of the present invention there is provided a pharmaceutical composition comprising:
a hydrophobic drug,
a digestible oil selected from triglycerides or propylene glycol esters of medium chain length (C₈-C₁₂) and/or long chain length (C₁₃-C₂₂) fatty acids; and propylene glycol monolaurate,
a lipophilic surfactant which comprises a glyceride of a C₅ to C₁₀ fatty acid and
a hydrophilic surfactant which is a polyoxyethylene hydrogenated castor oil,
wherein the digestible oil is present in an amount in the range from 3.0 to 12.0% by weight of the composition and the weight ratio of hydrophilic surfactant to lipophilic surfactant is in the range 1 : 1.5 to 1 : 2.5.

The formulations of the invention allow the drug to be completely solubilised in the liquid formulation. As a solution the drug is presented to the body in the most available form, avoiding the problems of slow disintegration and dissolution associated with other solid oral dosage formulations. This also obviates the need for micronised progesterone and subsequent control of the drug particle size. The formulations may be readily filled in hard or soft capsules.

The blend of digestible oil and surfactants used in the invention provides good solubilisation of the hydrophobic drug. The improved solvent power may be exploited by the formulators in various different ways. It is possible to employ a smaller capsule size to deliver the same drug dose compared with similar formulations in the prior art. Alternatively, or in addition, it is possible to reduce or eliminate ethanol and/or unsaturated compounds, such as maisine, which have been employed in prior art formulations to improve solubilisation of the drug.

The formulations of the invention have been designed to disperse immediately in aqueous environments such as the gastrointestinal tract, forming fine emulsions or microemulsions. In addition, the liquid excipients are chosen such that the emulsified formulation undergoes the natural rapid process of fat digestion (lipolysis). The submicroscopic mixed micelles formed by this process incorporate the products of vehicle lipolysis and solubilised drug. Solubilised drug leaves the microemulsion droplets, the vesicles and the micelles by diffusion. The surface area is vast so the diffusion process is very rapid. Any remaining solubilised drug in the micelles is released when the micelles deaggregate at the intestinal wall. Absorption of progesterone across the gastrointestinal wall in association with these mixed micelles is thought to contribute to the increase in drug bioavailability.

Hydrophobic drugs of interest in the present invention are sex hormones, particularly progesterone, oestradiol and testosterone. The invention allows formulations containing in excess of 5% by weight, preferably more than 6% by weight of progesterone in solution to be prepared which allows 50mg dose of progesterone to be encapsulated in softgel capsule, size 12 oblong which is considerably smaller than the size 20 oblong required to deliver 50mg of progesterone in the formulations disclosed in WO95/2493.

Furthermore, the invention allows ethanol to be completely eliminated from the formulations containing hydrophobic drugs. For example, an ethanol free formulation containing progesterone in solution and providing a 25mg dose in a softgel capsule may be formulated and filled into a size 9.5 oblong.

The reference to capsule sizes and shapes herein refer to softgel capsules. The capsule size provides an indication of the nominal fill volume (NFV). Examples of capsule sizes include:

| Capsule Size | NFV (minims) | NFV (cm³) |
|---|---|---|
| 4 | 3.0 - 4.0 | 0.185 - 0.246 |
| 9.5 | 7.5 - 9.5 | 0.462 - 0.585 |
| 20 | 16 - 20 | 0.986 - 1.232 |
| 10 | 7.5 - 10.0 | 0.462 - 0.616 |
| 18 | 15.0 - 18.0 | 0.924 - 1.109 |

The compositions of the invention employ smaller amounts of digestible oil than used in W092/24893. Generally 3 to 12%, preferably 4 to 7. The preferred digestible oil is fractionated coconut oil although other digestible oils, such as, peanut oil, soyabean oil, propylene glycol monolaurate and propylene glycol ester of fractionated coconut oil which is commercially available under the trade name Miglyol 840 may be used.

A blend of hydrophilic and lipophilic surfactants is present in the composition of the invention. The hydrophilic surfactant comprises a polyoxyethylene hydrogenated castor oil, preferably polyoxyethylene (40) hydrogenated castor oil, such as the product commercially available under the trade name Cremophor RH40. The lipophilic surfactant is preferably a mixture of glyceryl mono-and di-caprylate, such as the product commercially available under the trade name Imwitor 988. Other suitable lipophilic surfactants include a mixture of glyceryl mono- and dicaprates in combination with glyceryl mono- and di-caprylates. Such products are commercially available under the trade names Imwitor 742 and Capmul MCM.

The weight ratio of hydrophilic to lipophilic surfactant is important to achieve optimum solubilisation of the drugs. The weight ratio of hydrophilic to lipophilic surfactant is generally in the range from 1 : 1.5 to 1 : 2.5, usually 1 : 1.7 to 1 : 2.1. For progesterone and oestradiol formulations the ratio is preferably 1 : 1.80 to 1 : 1.90, most preferably about 1.85. Testosterone may be used in larger concentrations e.g. 8 to 16% by weight and the preferred weight ratio of hydrophilic to lipophilic surfactant is in the range 1 : 1.7 to 1 : 2.1.

The compositions may additionally comprise a co-solvent, such as ethanol. The presence of ethanol assists in increasing the concentration of drug which may be dissolved thereby allowing smaller volumes of formulation to achieve a desired unit dose. However, the presence of ethanol may result in an increased level of shell-fill interactions in capsules compared to formulations in which ethanol is absent. In addition ethanol can complicate the manufacturing process and packaging costs can increase where the final package must be impervious to ethanol. Formulations of the invention may be ethanol-free and still provide acceptable dosage levels of drug. The effective ratio of hydrophilic to lipophilic surfactant is not affected by the presence of ethanol.

The formulations of the invention do not require the presence of unsaturated components to solubilise the drug. Thus, compounds, such as Maisine 35-1, which could potentially react with other excipients or the drug itself, may be avoided. Additionally, unsaturated compounds could lead to cross-linking of the gelatin of capsules and ultimately a significantly increased disintegration time, possibly leading to poor adsorption. Preferably, the formulations are free from additives which are unsaturated compounds.

Suitable progesterone formulations containing ethanol in accordance with the invention comprise:
at least 5% by weight of progesterone
40 to 50% by weight of lipophilic surfactants
20 to 30% by weight of hydrophilic surfactants
3 to 10% by weight of digestible oil
15 to 25% by weight of ethanol.

Preferred formulations comprise:
5.5 to 6.5% by weight or progesterone
43 to 45% by weight of lipophilic surfactants
23 to 25% by weight of hydrophilic surfactants
4 to 9 by weight of digestible oil
18 to 20% by weight of ethanol.

A particularly preferred formulation comprises about:
6 parts by weight of progesterone
45 parts by weight of lipophilic surfactants
24 parts by weight of hydrophilic surfactants
4.5 parts by weight of digestible oil
20 parts by weight of ethanol.

Suitable ethanol-free formulations in accordance with the invention comprise a pharmaceutical composition comprising:
from 4 to 5% by weight of progesterone
55 to 60% by weight of lipophilic surfactants
30 to 35% by weight of hydrophilic surfactants
3 to 10% by weight of digestible oil.

A preferred formulation comprises about:
4.5 parts by weight of progesterone
58 parts by weight of lipophilic surfactants
31.5 parts by weight of hydrophilic surfactants
6 parts by weight of digestible oil.

The ethanol-containing and ethanol-free progesterone formulations may additionally comprise from 0.02 to 0.4% oestradiol without substantially altering the ratio of the other components.

The invention also provides ethanol-containing oestradiol formulations comprising:
0.05 to 2% by weight of oestradiol
45 to 50% by weight of lipophilic surfactants
22 to 27% by weight of hydrophilic surfactants
3 to 10% by weight of digestible oil
15 to 25% by weight of ethanol.

A preferred oestradiol formulation comprises about:
1 part by weight of oestradiol
48 parts by weight of lipophilic surfactants
26 parts by weight of hydrophilic surfactants
5 parts by weight of digestible oil
21 parts by weight of ethanol.

Ethanol-free oestradiol formulation in accordance with the invention comprise:
0.05 to 2% by weight of oestradiol
58 to 62% by weight of lipophilic surfactants
30 to 35% by weight of hydrophilic surfactants
5 to 7% by weight of digestible oil.

A preferred oestradiol formulation comprises about:
1 part by weight oestradiol
60 parts by weight of lipophilic surfactants
33 parts by weight of hydrophilic surfactants
6 parts by weight of digestible oil.

Suitable testosterone formulations in accordance with the invention comprise:
4 to 18% by weight of testosterone
40 to 48% by weight of lipophilic surfactants
20 to 25% by weight of hydrophilic surfactants
7 to 10% by weight of digestible oil
about 15% by weight of ethanol.

The formulations of the invention may comprise minor amounts of other components e.g. antioxidants.

In all of the above formulations the preferred components are fractionated coconut oil, Imwitor 988 and Cremophor RH40.

The formulation of the invention spontaneously form microemulsions when contacted with aqueous media and maintain the benefits of the composition disclosed in W095/24893 maintaining lipolysis and bioavailability of the drug.

The composition of the invention may readily be prepared by known methods, such as described in W095/24893. The compositions may be encapsulated in softgel or hardshell capsules. Methods of softgel encapsulation are disclosed in Theory and Practice of Industrial Pharmacy - Lachman & Leibermann, 2nd Edition, published by Henry Kimpton Publishers, London. Methods of liquid-fill hardshell encapsulation are disclosed in Hardcapsules - Development and Technology - Edited by K. Ridgeway, published by Pharmaceutical Press 1987.

The invention will now be illustrated by the following Examples.

The formulations reported in the following Tables in which all figures are in parts by weight were prepared.

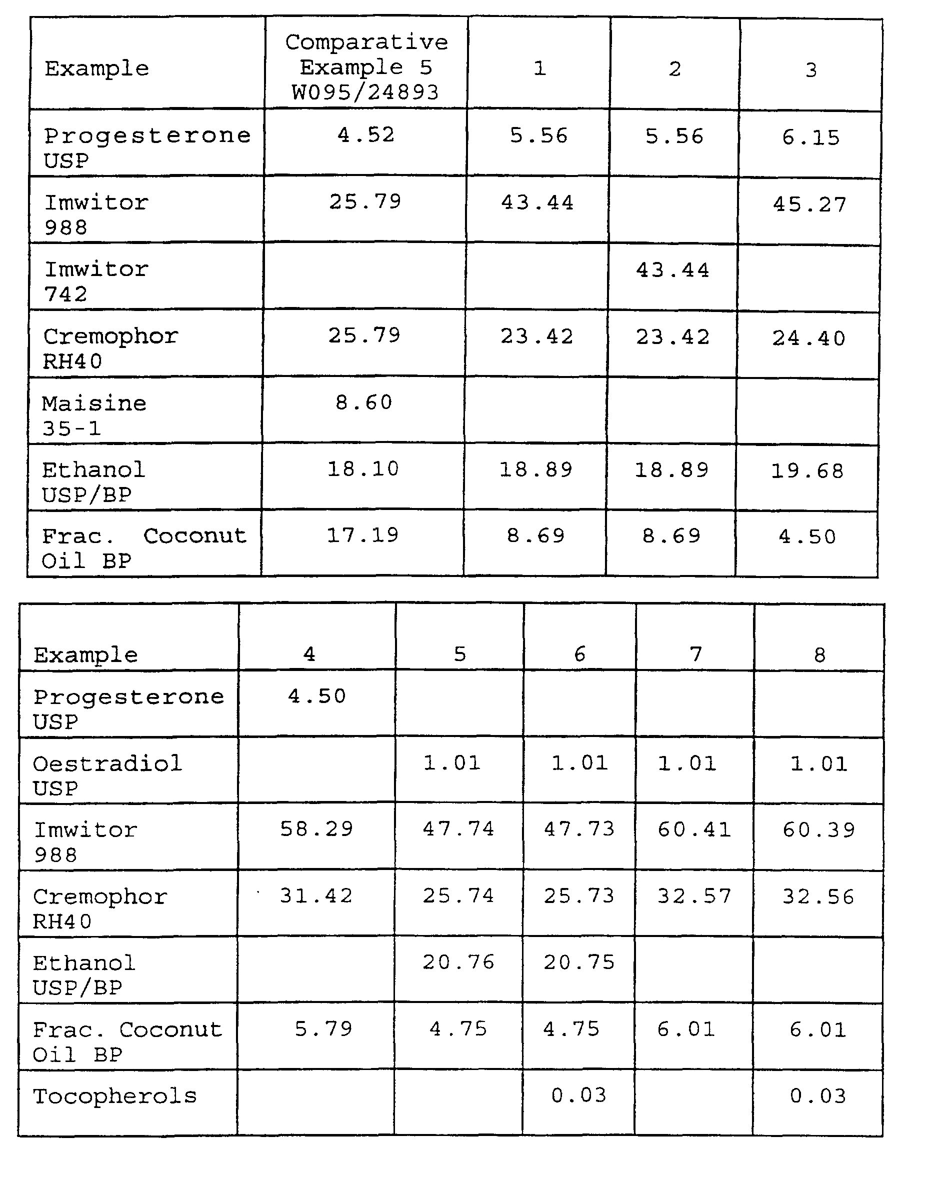

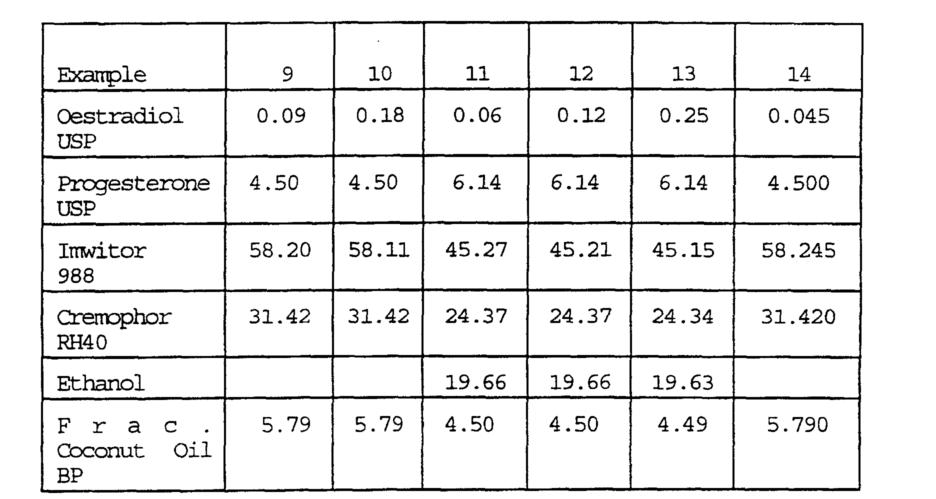

The formulation of each Example comprised stable, solutions of the drug.

The formulations may be filled into softgel capsules to provide a dosage form as follows:

| Example | Dose/Capsule | Softgel Capsule Size |
|---|---|---|
| Comparative | 50mg progesterone | 20 oblong |
| 1 | 50mg progesterone | 12 oblong |
| 2 | 50mg progesterone | 12 oblong |
| 3 | 50mg progesterone | 12 oblong |
| 4 | 25mg progesterone 50mg progesterone | 9.5 oblong or 10 oval 18 oblong |
| 5 | 2mg oestradiol | 4 oblong |
| 6 | 2mg oestradiol | 4 oblong |
| 7 | 2mg oestradiol | 4 oblong |
| 8 | 2mg oestradiol | 4 oblong |
| 9 | 0.5mg oestradiol 25mg progesterone 1mg oestradiol 50mg progesterone | 9.5 oblong or 10 oval 18 oblong |
| 10 | 1mg oestradiol 25mg progesterone | 9.5 oblong or 10 oval |
| 11 | 0.5mg oestradiol 50mg progesterone | 12 oblong |
| 12 | 1mg oestradiol 50mg progesterone | 12 oblong |
| 13 | 2mg oestradiol 50mg progesterone | 12 oblong |
| 14 | 0.5mg oestradiol 50mg progesterone 0.25mg oestradiol 25mg progesterone | 18 oblong 9.5 oblong or 10 oval |

### Relative Rates of Lipolysis

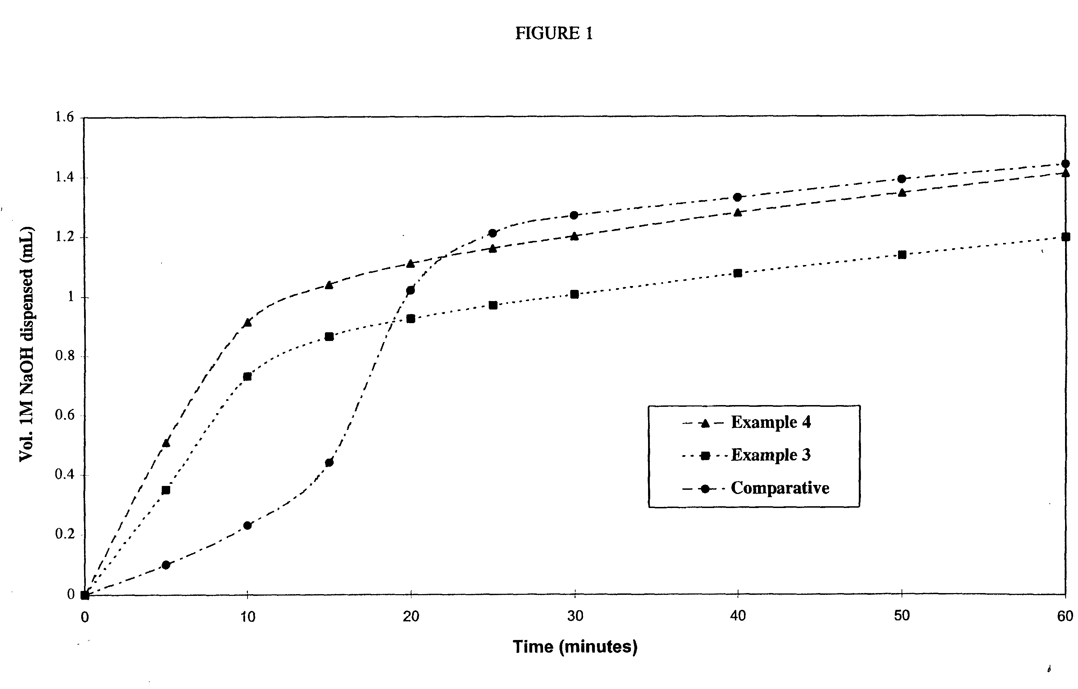

The relative rates of lipolysis for formulations of the Comparative Example and Examples 3 and 4 were measured in accordance with the in vitro test procedure described in W095/24893. The results are reported in Figure 1 of the accompanying drawings which represents a plot of NaOH dispensed against time for the formulations. The composition of the invention, both with and without ethanol indicate effective lipolysis is maintained.

### Bioavailability Study

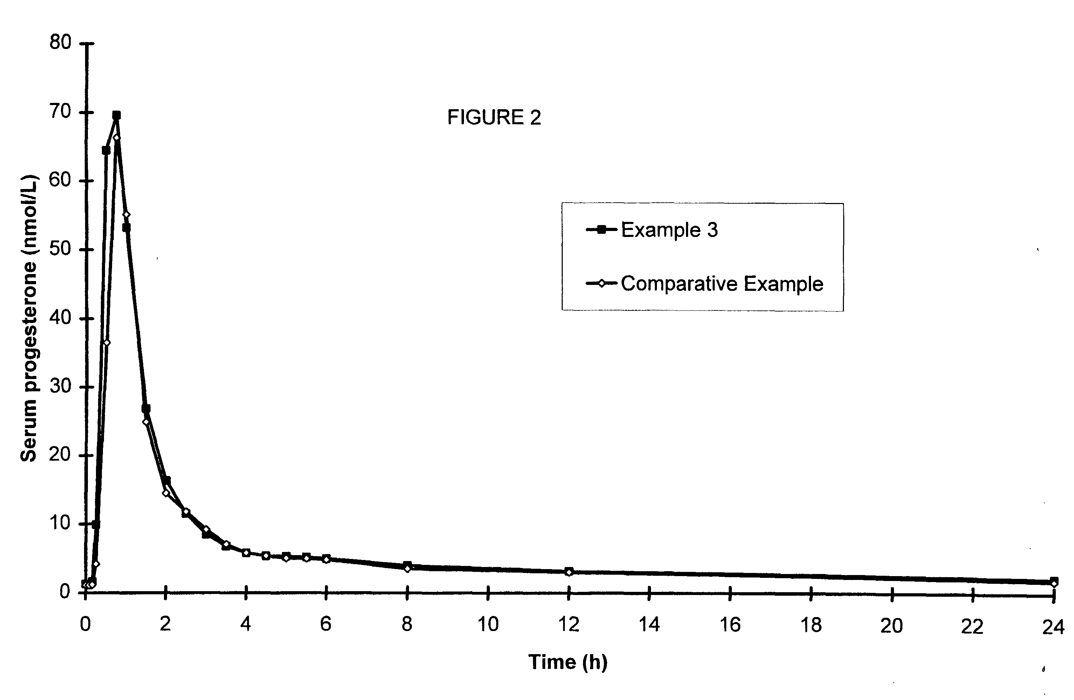

The bioavailability of progesterone from the formulations of the Comparative Example delivered in a 20 oblong softgel capsule and Example 3 delivered in a 12 oblong softgel capsule was measured. The results of the study are reported in Figure 2 of the accompanying drawings which represent a plot of mean serum concentration of progesterone against time for following the administration to 12 subjects of a single oral dose of each softgel capsule containing the progesterone. It will be seen the bioavailability of the formulation of the invention is substantially identical to the Comparative Example.

### Examples 15 to 20

The oestradiol formulation reported in the following Table were prepared in which all figures are in parts by weight. All formulations were in the form of solutions of oestradiol.

| Example | 15 | 16 | 17 | 18 | 19 | 20 |
|---|---|---|---|---|---|---|
| Oestradiol USP | 0.047 | 0.094 | 0.188 | 0.033 | 0.066 | 0.132 |
| Imwitor 988 | 60.993 | 60.946 | 60.852 | 48.197 | 48.164 | 48.098 |
| Cremophor RH40 | 32.900 | 32.900 | 32.900 | 26.000 | 26.000 | 26.000 |
| Ethanol USP/BP | | | | 20.970 | 20.970 | 20.970 |
| Frac. Coconut Oil BP | 6.060 | 6.060 | 6.060 | 4.800 | 4.800 | 4.800 |

The formulations were filled into softgel capsules as follows:

Example 15 0.25mg oestradiol (fill weight of 531mg) in 9.5 oblong or 10 oval capsule.

Example 16 0.5mg oestradiol (fill weight of 531mg) in 9.5 oblong or 10 oval capsule.

Example 17 1.0mg oestradiol (fill weight of 531mg) in 9.5 oblong or 10 oval capsule.

Example 18 0.25mg oestradiol (fill weight of 763mg) in 12 oblong capsule.

Example 19 0.5mg oestradiol (fill weight of 763mg) in 12 oblong capsule.

Example 20 1.0mg oestradiol (fill weight of 763mg) in 12 oblong capsule.

### Examples 21 to 38

The following Table, in which all figures are in parts by weight, illustrate testosterone formulations in accordance with the invention. Examples 21 to 26 may be encapsulated to provide a dose of 20mg, Examples 27 to 32 may be encapsulated to provide a dose of 40mg and Examples 33 to 38 may be encapsulated to provide a dose of 80mg.

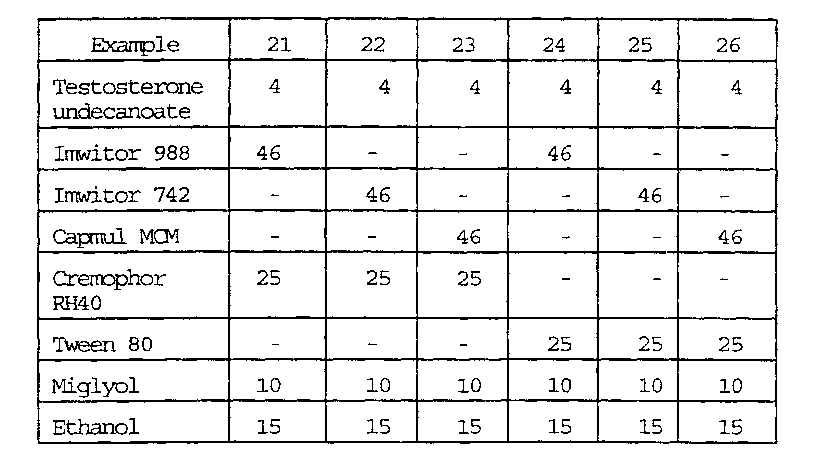

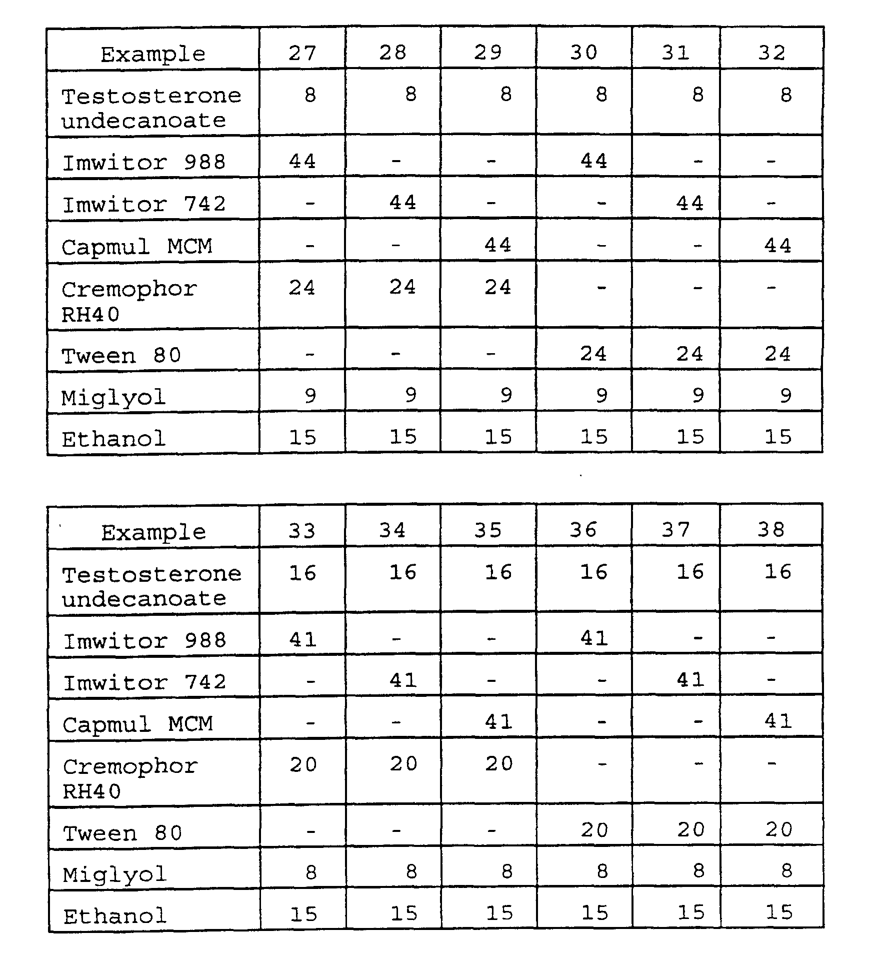

## Claims

1. A pharmaceutical composition comprising:
a hydrophobic drug,
a digestible oil selected from triglycerides or propylene glycol esters of medium chain length (C₈-C₁₂) and/or long chain length (C₁₃-C₂₂) fatty acids; and propylene glycol monolaurate,
a lipophilic surfactant which comprises a glyceride of a C₅ to C₁₀ fatty acid and
a hydrophilic surfactant which is a polyoxyethylene hydrogenated castor oil, wherein the digestible oil is present in an amount in the range from 3.0 to 12.0% by weight of the composition and the weight ratio of hydrophilic surfactant to lipophilic surfactant is in the range 1 : 1.5 to 1 : 2.5.

2. A pharmaceutical composition as claimed in Claim 1 in which the hydrophobic drug is dissolved and is selected from progesterone, oestradiol, testosterone and mixture of progesterone and oestradiol.

3. A pharmaceutical composition comprising:
at least 5% by weight of progesterone
40 to 50% by weight of lipophilic surfactants
20 to 30% by weight of hydrophilic surfactants
3 to 10% by weight of digestible oil
15 to 25% by weight of ethanol,
in which the weight ratio of hydrophilic surfactant to lipophilic surfactant is in the range 1 : 1.5 to 1 : 2.5.

4. A pharmaceutical composition as claimed in Claim 3 comprising:
5.5 to 6.5% by weight of progesterone
43 to 45% by weight of lipophilic surfactants
23 to 25% by weight of hydrophilic surfactants
4 to 9% by weight of digestible oil
18 to 20% by weight of ethanol.

5. A pharmaceutical composition as claimed in Claim 4 comprising :
6 parts by weight of progesterone
45 parts by weight of lipophilic surfactants
24 parts by weight of hydrophilic surfactants
4.5 parts by weight of digestible oil
20 parts by weight of ethanol.

6. A pharmaceutical composition comprising:
4 to 5% by weight of progesterone
55 to 60% by weight of lipophilic surfactants
30 to 35% by weight of hydrophilic surfactants
3 to 10% by weight of digestible oil.

7. A pharmaceutical composition as claimed in Claim 6 comprising :
4.5 parts by weight of progesterone
58 parts by weight of lipophilic surfactants
31.5% parts by weight of hydrophilic surfactants
6% parts by weight of digestible oil.

8. A pharmaceutical composition as claimed in any one of Claims 3 to 7 which additionally comprises from 0.02 to 2.0% oestradiol.

9. A pharmaceutical composition comprising:
0.05 to 2% by weight of oestradiol
45 to 50% by weight of lipophilic surfactants
22 to 27% by weight of hydrophilic surfactants
3 to 10% by weight of digestible oil
15 to 25% by weight of ethanol.

10. A pharmaceutical composition as claimed in Claim 9 comprising :
1 part by weight of oestradiol
48 parts by weight of lipophilic surfactants
26 parts by weight of hydrophilic surfactants
5 parts by weight of digestible oil
21 parts by weight of ethanol.

11. A pharmaceutical composition comprising:
0.05 to 2% by weight of oestradiol
58 to 62% by weight of lipophilic surfactants
30 to 35% by weight of hydrophilic surfactants
5 to 7% by weight of digestible oil.

12. A pharmaceutical composition as claimed in Claim 11 comprising :
1 part by weight oestradiol
60 parts by weight of lipophilic surfactants
33 parts by weight of hydrophilic surfactants
6 parts by weight of digestible oil.

13. A pharmaceutical composition comprising:
4 to 18% by weight of testosterone
40 to 48% by weight of lipophilic surfactants
20 to 25% by weight of hydrophilic surfactants
7 to 10% by weight of digestible oil
about 15% by weight of ethanol.

14. A pharmaceutical composition as claimed in any preceding claim in which the digestible oil is fractionated coconut oil.

15. A pharmaceutical composition as claimed in any preceding Claim in which the lipophilic surfactant comprises a mixture of glyceryl mono- and di-caprylate.

16. A pharmaceutical composition as claimed in Claim 15 in which the lipophilic surfactant additionally comprises a mixture of glyceryl mono-and di-caprate.

17. A pharmaceutical composition as claimed in any preceding Claim in which the hydrophilic surfactant comprises polyoxyethylene (40) hydrogenated castor oil.

18. A pharmaceutical composition as claimed in Claim 1 or Claim 2 which additionally comprises up to 25% by weight of the composition of ethanol.

19. A pharmaceutical composition as claimed in any preceding Claim in which the weight ratio of hydrophilic surfactants to lipophilic surfactants is in the range 1 : 1.5 to 1 : 2.5.

20. A pharmaceutical composition as claimed in Claim 18 in which the weight ratio of hydrophilic surfactants to lipophilic surfactants is in the range 1 : 1.80 to 1 : 1.90.

21. A pharmaceutical composition as claimed in Claim 19 in which the weight ratio of hydrophilic surfactants to lipophilic surfactants is 1 : 1.85.

22. A pharmaceutical composition as claimed in Claim 1 or Claim 2 which is free of ethanol.

23. A pharmaceutical composition as claimed in Claim 1 or Claim 2 which is free of additives which are unsaturated compounds.

24. A hard or soft capsule filled with a pharmaceutical composition as claimed in any preceding Claim.

25. A softgel capsule as claimed in Claim 24 comprising a capsule of size 12 oblong containing 50mg of progesterone.

26. A softgel capsule as claimed in Claim 24 comprising a capsule of size 9.5 oblong or 10 oval containing 25mg progesterone in an ethanol-free formulation.

27. A softgel capsule as claimed in Claim 25 or Claim 26 in which the capsule additionally contains from 0.25 to 2mg of oestradiol.

28. A softgel capsule as claimed in Claim 24 containing from 20 to 80mg testosterone.

## Patentansprüche

1. Pharmazeutische Zusammensetzung, aufweisend:
ein hydrophobes Arzneimittel,
ein verdauliches Öl, ausgewählt aus Triglyceriden oder Propylenglykolestern von Fettsäuren mittlerer Kettenlänge (C₈-C₁₂) und/oder langer Kettenlänge (C₁₃-C₂₂), sowie Propylenglykolmonolaurat;
ein lipophiles Tensid, das ein Glycerid einer C₅- bis C₁₀-Fettsäure aufweist, sowie
ein hydrophiles Tensid, das ein Polyoxyethylen-hydriertes Rizinusöl ist;
wobei das verdauliche Öl in einer Menge im Bereich von 3,0 bis 12,0 Gewichtsprozent der Zusammensetzung vorliegt und das Gewichtsverhältnis von hydrophilem Tensid zu lipophilem Tensid im Bereich von 1:1,5 bis 1:2,5 liegt.

2. Pharmazeutische Zusammensetzung nach Anspruch 1, bei welcher das hydrophobe Arzneimittel gelöst ist und ausgewählt ist aus Progesteron, Östradiol, Testosteron und Mischung von Progesteron und Östradiol.

3. Pharmazeutische Zusammensetzung, aufweisend:
mindestens 5 Gewichtsprozent Progesteron,
40 bis 50 Gewichtsprozent lipophile Tenside,
20 bis 30 Gewichtsprozent hydrophile Tenside,
3 bis 10 Gewichtsprozent verdauliches Öl,
15 bis 25 Gewichtsprozent Ethanol,
wobei das Gewichtsverhältnis von hydrophilem Tensid zu lipophilem Tensid im Bereich von 1:1,5 bis 1:2,5 liegt.

4. Pharmazeutische Zusammensetzung nach Anspruch 3, aufweisend:
5,5 bis 6,5 Gewichtsprozent Progesteron,
43 bis 45 Gewichtsprozent lipophile Tenside,
23 bis 25 Gewichtsprozent hydrophile Tenside,
4 bis 9 Gewichtsprozent verdauliches Öl,
18 bis 20 Gewichtsprozent Ethanol.

5. Pharmazeutische Zusammensetzung nach Anspruch 4, aufweisend:
6 Gewichtsteile Progesteron,
45 Gewichtsteile lipophile Tenside,
24 Gewichtsteile hydrophile Tenside,
4,5 Gewichtsteile verdauliches Öl,
20 Gewichtsteile Ethanol.

6. Pharmazeutische Zusammensetzung, aufweisend:
4 bis 5 Gewichtsprozent Progesteron,
55 bis 60 Gewichtsprozent lipophile Tenside,
30 bis 35 Gewichtsprozent hydrophile Tenside,
3 bis 19 Gewichtsprozent verdauliches Öl.

7. Pharmazeutische Zusammensetzung nach Anspruch 6, aufweisend:
4,5 Gewichtsteile Progesteron,
58 Gewichtsteile lipophile Tenside,
31,5 Gewichtsteile hydrophile Tenside,
6 Gewichtsteile verdauliches Öl.

8. Pharmazeutische Zusammensetzung nach einem der vorgenannten Ansprüche 3 bis 7, welche zusätzlich 0,02 bis 2,0% Östradiol aufweist.

9. Pharmazeutische Zusammensetzung, aufweisend:
0,05 bis 2 Gewichtsprozent Östradiol,
45 bis 50 Gewichtsprozent lipophile Tenside,
22 bis 27 Gewichtsprozent hydrophile Tenside,
3 bis 10 Gewichtsprozent verdauliches Öl,
15 bis 25 Gewichtsprozent Ethanol.

10. Pharmazeutische Zusammensetzung nach Anspruch 9, aufweisend:
1 Gewichtsteil Östradiol,
48 Gewichtsteile lipophile Tenside,
26 Gewichtsteile hydrophile Tenside,
5 Gewichtsteile verdauliches Öl,
21 Gewichtsteile Ethanol.

11. Pharmazeutische Zusammensetzung, aufweisend:
0,05 bis 2 Gewichtsprozent Östradiol,
58 bis 62 Gewichtsprozent lipophile Tenside,
30 bis 35 Gewichtsprozent hydrophile Tenside,
5 bis 7 Gewichtsprozent verdauliches Öl.

12. Pharmazeutische Zusammensetzung nach Anspruch 11, aufweisend:
1 Gewichtsteil Östradiol,
60 Gewichtsteile lipophile Tenside,
33 Gewichtsteile hydrophile Tenside,
6 Gewichtsteile verdauliches Öl.

13. Pharmazeutische Zusammensetzung, aufweisend:
4 bis 18 Gewichtsprozent Testosteron,
40 bis 48 Gewichtsprozent lipophile Tenside,
20 bis 25 Gewichtsprozent hydrophile Tenside,
7 bis 10 Gewichtsprozent verdauliches Öl,
etwa 15 Gewichtsprozent Ethanol.

14. Pharmazeutische Zusammensetzung nach einem der vorgenannten Ansprüche, bei welcher das verdauliche Öl fraktioniertes Kokosnussöl ist.

15. Pharmazeutische Zusammensetzung nach einem der vorgenannten Ansprüche, bei welcher das lipophile Tensid eine Mischung von Glycerylmono- und Glyceryldicaprylat aufweist.

16. Pharmazeutische Zusammensetzung nach Anspruch 15, bei welcher das lipophile Tensid zusätzlich Mischung von Glycerylmono- und Glyceryldicaprylat aufweist.

17. Pharmazeutische Zusammensetzung nach einem der vorgenannten Ansprüche, bei welcher das hydrophile Tensid Polyoxyethylen(40)-hydriertes Rizinusöl aufweist.

18. Pharmazeutische Zusammensetzung nach Anspruch 1 oder 2, die zusätzlich bis zu 25 Gewichtsprozent der Zusammensetzung Ethanol aufweist.

19. Pharmazeutische Zusammensetzung nach einem der vorgenannten Ansprüche, bei welcher das Gewichtsverhältnis von hydrophilen Tensiden zu lipophilen Tensiden im Bereich von 1:1,5 bis 1:2,5 liegt.

20. Pharmazeutische Zusammensetzung nach Anspruch 18, bei welcher das Gewichtsverhältnis von hydrophilen Tensiden zu lipophilen Tensiden im Bereich von 1:1,80 bis 1:1,90 liegt.

21. Pharmazeutische Zusammensetzung nach Anspruch 19, bei welcher das Gewichtsverhältnis von hydrophilen Tensiden zu lipophilen Tensiden im Bereich von 1:1,85 liegt.

22. Pharmazeutische Zusammensetzung nach Anspruch 1 oder 2, die frei ist von Ethanol.

23. Pharmazeutische Zusammensetzung nach Anspruch 1 oder 2, die frei ist von Additiven, die ungesättigte Verbindungen sind.

24. Hartkapsel oder Weichkapsel, die gefüllt ist mit einer pharmazeutischen Zusammensetzung nach einem der vorgenannten Ansprüche.

25. Weichgelkapsel nach Anspruch 24, aufweisend eine Kapsel der Größe 12 Oblong, die 50 mg Progesteron enthält.

26. Weichgelkapsel nach Anspruch 24, aufweisend eine Kapsel der Größe 9,5 Oblong oder 10 Oval, die 25 mg Progesteron in einer ethanolfreien Zubereitung enthält.

27. Weichgelkapsel nach Anspruch 25 oder 26, bei welcher die Kapsel zusätzlich 0,25 bis 2 mg Östradiol enthält.

28. Weichgelkapsel nach Anspruch 24, enthaltend 20 bis 80 mg Testosteron.

## Revendications

1. Composition pharmaceutique comprenant:
un médicament hydrophobe,
une huile digestible choisie parmi les triglycérides ou les esters de propylèneglycol et d'acides gras à longueur de chaîne moyenne (C₈ à C₁₂) et/ou à longueur de chaîne longue (C₁₃ à C₂₂); et le monolaurate de propylèneglycol,
un agent tensioactif lipophile qui comprend un glycéride d'un acide gras en C₅ à C₁₀ et
un agent tensioactif hydrophile qui est une huile de ricin hydrogénée polyoxyéthylénée,
dans laquelle l'huile digestible est présente en une quantité dans la gamme de 3,0 à 12,0% en poids de la composition et le rapport pondéral de l'agent tensioactif hydrophile à l'agent tensioactif lipophile est dans la gamme de 1:1,5 à 1:2,5.

2. Composition pharmaceutique selon la revendication 1, dans laquelle le médicament hydrophobe est dissous et est choisi parmi la progestérone, l'oestradiol, la testostérone et un mélange de progestérone et d'oestradiol.

3. Composition pharmaceutique comprenant:
au moins 5% en poids de progestérone
40 à 50% en poids d'agents tensioactifs lipophiles
20 à 30% en poids d'agents tensioactifs hydrophiles
3 à 10% en poids d'huile digestible
15 à 25% en poids d'éthanol,
dans laquelle le rapport pondéral de l'agent tensioactif hydrophile à l'agent tensioactif lipophile est dans la gamme de 1:1,5 à 1:2,5.

4. Composition pharmaceutique selon la revendication 3, comprenant:
5,5 à 6,5% en poids de progestérone
43 à 45% en poids d'agents tensioactifs lipophiles
23 à 25% en poids d'agents tensioactifs hydrophiles
4 à 9% en poids d'huile digestible
18 à 20% en poids d'éthanol.

5. Composition pharmaceutique selon la revendication 4, comprenant:
6 parties en poids de progestérone
45 parties en poids d'agents tensioactifs lipophiles
24 parties en poids d'agents tensioactifs hydrophiles
4,5 parties en poids d'huile digestible
20 parties en poids d'éthanol.

6. Composition pharmaceutique comprenant:
4 à 5% en poids de progestérone
55 à 60% en poids d'agents tensioactifs lipophiles
30 à 35% en poids d'agents tensioactifs hydrophiles
3 à 10% en poids d'huile digestible.

7. Composition pharmaceutique selon la revendication 6, comprenant:
4,5 parties en poids de progestérone
58 parties en poids d'agents tensioactifs lipophiles
31,5 parties en poids d'agents tensioactifs hydrophiles
6 parties en poids d'huile digestible.

8. Composition pharmaceutique selon l'une quelconque des revendications 3 à 7, qui comprend en plus de 0,02 à 2,0% d'oestradiol.

9. Composition pharmaceutique comprenant:
0,05 à 2% en poids d'oestradiol
45 à 50% en poids d'agents tensioactifs lipophiles
22 à 27% en poids d'agents tensioactifs hydrophiles
3 à 10% en poids d'huile digestible
15 à 25% en poids d'éthanol.

10. Composition pharmaceutique selon la revendication 9, comprenant:
1 partie en poids d'oestradiol
48 parties en poids d'agents tensioactifs lipophiles
26 parties en poids d'agents tensioactifs hydrophiles
5 parties en poids d'huile digestible
21 parties en poids d'éthanol.

11. Composition pharmaceutique comprenant:
0,05 à 2% en poids d'oestradiol
58 à 62% en poids d'agents tensioactifs lipophiles
30 à 35% en poids d'agents tensioactifs hydrophiles
5 à 7% en poids d'huile digestible.

12. Composition pharmaceutique selon la revendication 11, comprenant:
1 partie en poids d'oestradiol
60 parties en poids d'agents tensioactifs lipophiles
33 parties en poids d'agents tensioactifs hydrophiles
6 parties en poids d'huile digestible.

13. Composition pharmaceutique comprenant:
4 à 18% en poids de testostérone
40 à 48% en poids d'agents tensioactifs lipophiles
20 à 25% en poids d'agents tensioactifs hydrophiles
7 à 10% en poids d'huile digestible
environ 15% en poids d'éthanol.

14. Composition pharmaceutique selon l'une quelconque des revendications précédentes, dans laquelle l'huile digestible est de l'huile de coco fractionnée.

15. Composition pharmaceutique selon l'une quelconque des revendications précédentes, dans laquelle l'agent tensioactif lipophile comprend un mélange de mono- et de dicaprylate de glycéryle.

16. Composition pharmaceutique selon la revendication 15, dans laquelle l'agent tensioactif lipophile comprend en plus un mélange de mono- et de dicaprate de glycéryle.

17. Composition pharmaceutique selon l'une quelconque des revendications précédentes, dans laquelle l'agent tensioactif hydrophile comprend de l'huile de ricin hydrogénée polyoxyéthylénée (40).

18. Composition pharmaceutique selon la revendication 1 ou la revendication 2, qui comprend en plus jusqu'à 25% en poids d'éthanol par rapport à la composition.

19. Composition pharmaceutique selon l'une quelconque des revendications précédentes, dans laquelle le rapport pondéral des agents tensioactifs hydrophiles aux agents tensioactifs lipophiles est dans la gamme de 1:1,5 à 1:2,5.

20. Composition pharmaceutique selon la revendication 18, dans laquelle le rapport pondéral des agents tensioactifs hydrophiles aux agents tensioactifs lipophiles est dans la gamme de 1:1,80 à 1:1,90.

21. Composition pharmaceutique selon la revendication 19, dans laquelle le rapport pondéral des agents tensioactifs hydrophiles aux agents tensioactifs lipophiles est de 1:1,85.

22. Composition pharmaceutique selon la revendication 1 ou la revendication 2, qui est exempte d'éthanol.

23. Composition pharmaceutique selon la revendication 1 ou la revendication 2, qui est exempte d'additifs qui sont des composés insaturés.

24. Capsule dure ou molle remplie avec une composition pharmaceutique selon l'une quelconque des revendications précédentes.

25. Capsule en gel mou selon la revendication 24, comprenant une capsule de taille 12 oblongue contenant 50 mg de progestérone.

26. Capsule en gel mou selon la revendication 24, comprenant une capsule de taille 9,5 oblongue ou 10 ovale contenant 25 mg de progestérone dans une formulation exempte d'éthanol.

27. Capsule en gel mou selon la revendication 25 ou la revendication 26, dans laquelle la capsule contient en plus de 0,25 à 2 mg d'oestradiol.

28. Capsule en gel mou selon la revendication 24, contenant de 20 à 80 mg de testostérone.
